# EUROPEAN PATENT APPLICATION

(11) **EP 4 610 244 A1**
(43) Date of publication of application: **03.09.2025**
(21) Application number: 23883052.5
(22) Date of filing: 24.10.2023
(51) Int. Cl.: C07C 1/04, C07C 2/76, C07C 11/02, C07C 15/04, C07C 15/06, C07C 15/08

(54) **METHOD FOR PREPARING RAW MATERIAL FOR PLASTICS BY USING STEEL BY-PRODUCT GAS**

(30) Priority: 25.10.2022 KR 20220138032; 19.10.2023 KR 20230140475
(71) Applicant: Korea Research Institute of Chemical Technology, Daejeon 34114 (KR)
(72) Inventor: PARK, Yong Ki, Daejeon 34114 (KR); KIM, Ki Woong, Daejeon 34114 (KR); KANG, Na Young, Daejeon 34114 (KR); PARK, Jaedeuk, Daejeon 34114 (KR); PARK, Dae Sung, Daejeon 34114 (KR); SHIN, Ji Ho, Daejeon 34114 (KR); KIM, Do Kyoung, Daejeon 34114 (KR)
(74) Representative: Kraus & Lederer PartGmbB
(86) International application number: PCT/KR2023/016539
(87) International publication number: WO 2024/090946

(57) **Abstract**

The present invention relates to a method for preparing, from steel by-product gas, high-value-added olefins, which are used as a raw material for plastics. The method for preparing olefins by using steel by-product gas, according to the present invention, mainly comprises, as an integrated process, the steps of: (1) supplying a carbon monoxide-rich stream from steel by-product gas, and a hydrogen-rich stream from synthesis gas; (2) receiving carbon monoxide and hydrogen and converting same into an oxygenate; and (3) reacting the oxygenate with a petroleum-derived hydrocarbon simultaneously, thereby producing light olefins, BTX and the like, which are raw materials for plastics.

## Description

### [Technical Field]

The present invention relates to a process of preparing a raw material for carbon reduction plastics by utilizing by-product gas generated in steel mills and the like.

The present invention relates to an integrated process of synthesizing an oxygenate from by-product gas and reacting the same with a petroleum-derived hydrocarbon to produce olefins, BTX, and the like, which may be used as raw materials for plastics.

In the present invention, since by-product gas having a large carbon dioxide emission coefficient is utilized, not only greenhouse gas emissions may be reduced, but also high-value-added olefins may be produced from carbon dioxide.

### [Background Art]

Active efforts are under way worldwide to reduce greenhouse gases such as carbon dioxide so as to cope with global warming. In Korea, carbon neutrality by 2050 has been declared and is being promoted across all social sectors.

Meanwhile, since by-product gas generated in steel mills has a high carbon dioxide content and causes a significant burden in greenhouse gas emissions, various efforts are currently being made to reuse the by-product gas for self-generation or processes, but most of the uses are still low-value-added.

In particular, blast furnace gas, among the types of steel by-product gas, not only has a high carbon dioxide content, but also accounts for approximately 80% of the carbon dioxide emitted from steel mills; thus, there is a need for a process of efficiently separating and recovering the blast furnace gas and converting the blast furnace gas into high-value-added gas. Korean Patent Laid-Open Publication No. 10-2016-0098339 describes that blast furnace top gas may be used to produce syngas, but only the concept is described without a separate separation process, and thus, the carbon dioxide removal technology and efficiency are unknown. In addition, Korean Patent No. 10-2258543 discloses a technology for separating gas components from blast furnace gas and using the gas components for urea synthesis; however, in the registered patent, carbon dioxide is removed using pressure swing adsorption, which is disadvantageous in terms of energy efficiency, and there is no description of reuse of the separated carbon dioxide.

Therefore, there is an urgent need to develop an integrated process for producing a raw material for plastics from steel by-product gas that may create economic added value while satisfying the social requirement of carbon neutrality.

### [Related Art Document]

### [Patent Documents]

(Patent Document 1) Korean Patent Laid-Open Publication No. 10-2016-0098339
(Patent Document 2) Korean Patent No. 10-1803406
(Patent Document 3) Korean Patent No. 10-2258543

### [Disclosure]

### [Technical Problem]

An object of the present invention is to provide a method for producing a carbon monoxide-rich stream by selectively separating and recovering carbon dioxide from steel by-product gas, and then supplying the carbon monoxide-rich stream together with hydrogen-rich gas to synthesize an oxygenate.

Specifically, one specific object of the present invention is to provide a step of separating carbon dioxide from steel by-product gas using a membrane contactor and supplying the separated carbon dioxide to a carbon capture and storage process.

Specifically, another specific object of the present invention is to provide a step of separating nitrogen and hydrogen using pressure swing adsorption after separating carbon dioxide, supplying the separated nitrogen to an ammonia production process, and reusing the separated hydrogen for synthesis of an oxygenate.

Another object of the present invention is to provide a method for producing a raw material for plastics by reacting a synthesized oxygenate with a petroleum-derived hydrocarbon simultaneously.

Still another object of the present invention is to provide a step for supplying a synthesized oxygenate to an olefin synthesis process simultaneously with a petroleum-derived hydrocarbon without a separate distillation process to produce high-value-added olefins.

### [Technical Solution]

A first aspect of the present invention provides a method for preparing a raw material for plastics from steel by-product gas, the method including the steps of: (1-1) separating carbon dioxide from a steel by-product gas stream to produce a carbon monoxide-rich (CO-rich) gas stream; (1-2) supplying a hydrogen-rich (H₂-rich) gas stream from syngas or renewable hydrogen; (2) receiving the carbon monoxide-rich gas stream in the step (1-1) and the hydrogen-rich gas stream in the step (1-2) and synthesizing an oxygenate; and (3) reacting the oxygenate synthesized in the step (2) with a petroleum-derived hydrocarbon simultaneously to produce olefins, wherein in the step (1-1), the carbon dioxide in the steel by-product gas is 1 to 50 vol% and is separated through a membrane contactor.

A second aspect of the present invention provides a method for preparing a raw material for plastics from steel by-product gas, the method including the steps of: (1-1) separating carbon dioxide from a steel by-product gas stream to produce a carbon monoxide-rich (CO-rich) gas stream; (1-2) supplying a hydrogen-rich (H2-rich) gas stream from syngas or renewable hydrogen; (2) receiving the carbon monoxide-rich gas stream in the step (1-1) and the hydrogen-rich gas stream in the step (1-2) and synthesizing an oxygenate; and (3) reacting the oxygenate synthesized in the step (2) with a petroleum-derived hydrocarbon simultaneously to produce olefins, wherein the oxygenate synthesized in the step (2) is introduced into the step (3) without a separate purification process to continuously produce olefins.

In the method for preparing a raw material for plastics from steel by-product gas according to one specific example of the present invention, the steel by-product gas may be blast furnace gas (BFG), and may include 10 to 30 vol% of carbon dioxide, 10 to 30 vol% of carbon monoxide, 40 to 70 vol% of nitrogen, and 1 to 5 vol% of hydrogen.

In the method for preparing a raw material for plastics according to one specific example of the present invention, the oxygenate may be an alcohol, an ether, or a mixture thereof.

In the method for preparing a raw material for plastics according to one specific example of the present invention, the petroleum-derived hydrocarbon may include one or more selected from naphtha, gasoline, kerosene, and diesel.

In the method for preparing a raw material for plastics according to one specific example of the present invention, the method may further include, after the separation of the carbon dioxide in the step (1-1), a step of sequentially separating nitrogen and hydrogen or sequentially separating hydrogen and nitrogen.

In the method for preparing a raw material for plastics according to one specific example of the present invention, in the step of separating hydrogen, a hydrogen membrane may be used.

In the method for preparing a raw material for plastics according to one specific example of the present invention, in the step of separating nitrogen, pressure swing adsorption (PSA) may be used.

In the method for preparing a raw material for plastics according to one specific example of the present invention, the separated hydrogen may be additionally mixed into the hydrogen-rich (H₂-rich) gas stream in the step (1-2) and reused.

In the method for preparing a raw material for plastics according to one specific example of the present invention, a molar ratio of H₂/(2CO+3CO₂) for methanol synthesis in the step (2) may be 0.9 to 1.5.

In the method for preparing a raw material for plastics according to one specific example of the present invention, the carbon dioxide separated from the steel by-product gas may be supplied to a carbon capture and storage (CCS) or carbon capture and utilization (CCU) process.

In the method for preparing a raw material for plastics according to one specific example of the present invention, the nitrogen separated by the pressure swing adsorption (PSA) may be supplied to a nitrogenate production process.

In the method for preparing a raw material for plastics according to one specific example of the present invention, in the step (3), a composite including the oxygenate may include 10 to 30 parts by weight of moisture (H₂O).

In the method for preparing a raw material for plastics according to one specific example of the present invention, in the step (3), a supply ratio of the oxygenate and the petroleum-derived hydrocarbon may be 0.7 to 5 parts by weight of the petroleum-derived hydrocarbon with respect to 1 part by weight of the oxygenate.

In the method for preparing a raw material for plastics according to one specific example of the present invention, reaction temperature and space velocity ranges in the step (3) may be 500 to 700°C and 5 to 30 h⁻¹.

### [Advantageous Effects]

According to the present invention, the synthesis of the oxygenate and the olefin production from the carbon monoxide-rich stream separated from the steel by-product gas may be performed as an integrated process.

According to the present invention, since carbon dioxide is separated using a membrane contactor, there is no pressure drop, and therefore, there is an advantage in reducing energy consumption.

In addition, according to the present invention, an oxygenate composite is reacted with a petroleum-derived hydrocarbon simultaneously in a crude state and used to produce olefins without removing moisture from the oxygenate composite, such that the process is simplified and the energy efficiency is high.

### [Description of Drawings]

FIG. 1 is a conceptual diagram of a process of preparing a raw material for plastics using steel by-product gas of the present invention.
FIG. 2 is a conceptual diagram of a process of preparing a raw material for plastics from steel by-product gas according to Example 1.
FIG. 3 is a conceptual diagram of a process of preparing a raw material for plastics from steel by-product gas according to Example 2.
FIG. 4 is a process conceptual diagram according to the presence or absence of a membrane contactor in Example 1 and Comparative Example 1.
FIG. 5 illustrates adsorption equilibrium experimental data of Example 1 and Comparative Example 1.
FIG. 6 is a process conceptual diagram according to the presence or absence of additional distillation purification of methanol in Example 1 and Comparative Example 2.
FIG. 7 is a comparative conceptual diagram of a conventional technology and an integrated process of the present invention for preparing a raw material for plastics from steel by-product gas.
FIG. 8 is a conceptual diagram of a process for each scenario for measuring a carbon dioxide reduction rate according to Example 3.

### [Best Mode]

Hereinafter, a method for preparing a raw material for plastics from steel by-product gas of the present invention will be described in detail with reference to the accompanying drawings. The features of the present invention and the method for achieving them will be clarified with reference to embodiments described in detail below together with the drawings to be described below. However, these embodiments are provided by way of example so that the spirit of the present invention may be sufficiently transferred. Therefore, the present invention may be implemented in different forms without being limited to the drawings to be described below, and the drawings to be described below may be exaggerated in order to specify the spirit of the present invention. In this case, unless otherwise defined, the technical terms and scientific terms used have the same meanings as commonly understood by those skilled in the art to which the present invention pertains. The description for the known function and configuration unnecessarily obscuring the gist of the present invention will be omitted in the following description and the accompanying drawings.

In addition, unless the context clearly indicates otherwise, singular forms used in the specification and the appended claims may be intended to include plural forms.

The terms "first", "second", and the like used in the present specification and the appended claims are not used for limitation but used to distinguish one component from another component.

The term "include(s)" or "have(has)" used in the present specification and the appended claims means that features or components described in the specification are present, and does not preclude the possibility that one or more other features or components may be added, unless specifically limited.

The present invention relates to a process of producing, from steel by-product gas, high-value-added olefins used as a raw material for plastics, and is characterized by including a process of separating carbon monoxide, carbon dioxide, and hydrogen included in steel by-product gas and preparing carbon monoxide-rich gas, a process of preparing hydrogen-rich gas from syngas or renewable hydrogen, a process of receiving carbon monoxide and hydrogen and synthesizing an oxygenate, and a process of reacting the synthesized oxygenate with a petroleum-derived hydrocarbon simultaneously to produce olefins.

A technology for preparing a raw material for carbon reduction plastics of the present invention mainly includes, as an integrated process, the steps of: (1) supplying a carbon monoxide-rich stream from steel by-product gas and a hydrogen-rich stream from syngas; (2) receiving carbon monoxide and hydrogen and converting the same into an oxygenate; and (3) reacting the oxygenate with a petroleum-derived hydrocarbon simultaneously to produce light olefins, BTX, and the like, which are raw materials for plastics.

The steps (1) and (2) may be called a "carbon reduction raw material utilization process" in terms of utilizing the steel by-product gas, and the step (3) may be called a "carbon reduction reaction/separation process".

The by-product gas generated in steel mills and the like of the present invention includes one or more types of by-product gas selected from the group consisting of blast furnace gas (BFG), Linz-Donawitz gas (LDG), coke oven gas (COG), and finex off gas (FOG).

BFG is a gas generated during the production of pig iron in a blast furnace, includes carbon dioxide, carbon monoxide, hydrogen, and nitrogen, and has a higher volume ratio of carbon dioxide compared to another by-product gas. The BFG components typically include 35 to 60 vol% of nitrogen, 20 to 30 vol% of carbon monoxide, 20 to 30 vol% of carbon dioxide, and 2 to 15 vol% of hydrogen in terms of a volume ratio.

LDG is a gas obtained in a converter steel mill, includes carbon dioxide, carbon monoxide, hydrogen, and nitrogen, and has a higher volume ratio of carbon monoxide. The Linz-Donawitz gas components include 50 to 70 vol% of carbon monoxide, 10 to 20 vol% of nitrogen, about 15 vol% of carbon dioxide, and about 2 vol% of hydrogen in terms of a volume ratio.

COG is a gas obtained during coking of coal, includes hydrogen, methane, nitrogen, carbon monoxide, and carbon dioxide, and has a high volume ratio of hydrogen. The Linz-Donawitz gas components typically include 55 to 70 vol% of hydrogen, 20 to 30 vol% of methane, 5 to 10 vol% of nitrogen, and 5 to 10 vol% of carbon monoxide in terms of a volume ratio, and additionally include carbon dioxide and the like.

The steel by-product gas that serves as a raw material supply source of the present invention typically includes carbon dioxide, carbon monoxide, nitrogen, and hydrogen, and is preferably BFG, which is generated in large quantities in the steel mill and has a relatively low calorific value per unit volume compared to another by-product gas, but is not limited thereto.

*

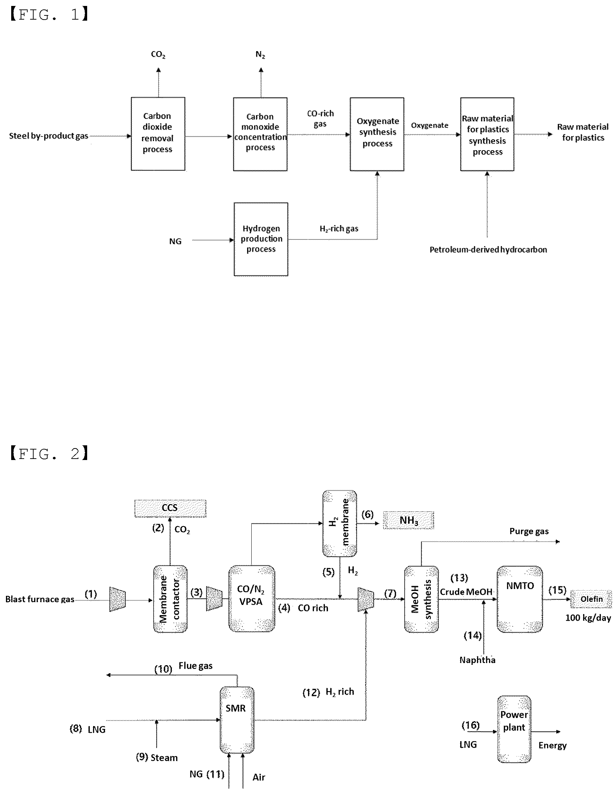

FIG. 1 illustrates a conceptual diagram of a process of preparing a raw material for plastics using steel by-product gas according to the present invention. As described above, the process of preparing a raw material for carbon reduction plastics of the present invention is an integrated form of a "carbon reduction raw material utilization process" and a "carbon reduction reaction/separation process".

Referring to FIG. 1, the oxygenate synthesis process includes the steps of:
(1-1) separating carbon dioxide from a steel by-product gas stream to produce a carbon monoxide-rich (CO-rich) gas stream; (1-2) supplying a hydrogen-rich (H₂-rich) gas stream from syngas or renewable hydrogen; (2) receiving the carbon monoxide-rich gas stream in the step (1-1) and the hydrogen-rich gas stream in the step (1-2) and synthesizing an oxygenate; and (3) reacting the oxygenate synthesized in the step (2) with a petroleum-derived hydrocarbon simultaneously to produce olefins.

According to the step (1-1) of the present invention, carbon dioxide, hydrogen, and nitrogen included in the steel by-product gas stream are separated and recovered to produce a carbon monoxide-rich gas stream, thereby obtaining a carbon monoxide-rich stream. There is no set order for separating each gas, but since the blast furnace gas has a high carbon dioxide concentration, it is preferable to separate carbon dioxide first and then separate hydrogen and carbon monoxide at a downstream so that hydrogen and carbon monoxide, which have relatively low concentrations, may be easily separated.

The process of separating carbon dioxide from the steel by-product gas stream according to the present invention includes one or more selected from an absorption method, a membrane contact method, an adsorption method, and a low-temperature distillation method. The process of separating carbon dioxide may preferably use a method using a membrane contactor, but is not limited thereto.

For the membrane contactor of the membrane contactor separation process, Korean Patent No. 10-1759101 by the same applicant may be referred to. Specifically, the membrane contactor separation process is a technology for bringing the steel by-product gas into contact with an absorbent using a porous hollow fiber membrane formed of a hydrophobic material, and then selectively absorbing and separating highly soluble carbon dioxide into the absorbent. Accordingly, the carbon dioxide separation process may be miniaturized due to its simple process, and has the advantages of low installation and operating costs.

The membrane contactor includes a housing; a hollow fiber membrane mounted in the housing; and an absorbent-filled space defined by an outer side of the hollow fiber membrane constituting the hollow fiber membrane and an inner side of the housing.

The hollow fiber membrane may include at least one selected from the group consisting of polytetrafluoroethylene (PTFE), polypropylene (PP), and polyvinylidene fluoride (PVDF).

The absorbent may include at least one selected from the group consisting of water, methanol, ethanol, dimethyl ethers of polyethylene glycol (DEPG), N-methyl-2-pyrrolidone (NMP), propylene carbonate (PC), sulfolane, methyl diethanolamine (MDEA), diethanolamine (DEA), methyl ethanolamine (MEA), and triethanolamine (TEA).

The carbon dioxide separation process using the membrane contactor may include the steps of: supplying an absorbent to the absorbent-filled space of the absorption module (membrane contactor); injecting steel by-product gas including carbon dioxide into the hollow fiber of the hollow fiber membrane; controlling a pressure in the absorption module so that carbon dioxide in the steel by-product gas may be selectively dissolved in the absorbent; and separating and discharging each of the absorbent including the carbon dioxide dissolved in the absorbent-filled space and gas containing relatively little carbon dioxide inside the hollow fiber to the outside of the absorption module.

In one specific example, when carbon dioxide is recovered using the membrane contactor, a recovery rate of carbon dioxide is 70% or more, preferably 80% or more, and more preferably 90% or more, and a purity of the recovered carbon dioxide is 80% or more, preferably 90% or more, and more preferably 95% or more.

The stream from which the carbon dioxide is separated according to the present invention is subjected to a step of separating nitrogen and carbon monoxide to produce a carbon monoxide-rich gas stream. The process of separating carbon monoxide includes one or more selected from pressure swing adsorption (PSA), an absorption method, a low-temperature distillation method, and a membrane separation method. Since blast furnace gas among the types of by-product gas of a steel mill includes about 54% of nitrogen, it is preferable to use a pressure swing adsorption (PSA) process, but it is not limited thereto.

In one specific example, in the carbon monoxide pressure swing adsorption process, impurities may be removed at an upstream of the process to increase the carbon monoxide recovery rate, and operating conditions of the pressure swing adsorption determined in advance may be utilized for a concentration ratio of input carbon monoxide and nitrogen. When the pressure swing adsorption process is used, a recovery rate of carbon monoxide is 70% or more, preferably 80% or more, and more preferably 90% or more, and a purity of the recovered carbon monoxide is 90% or more, preferably 95% or more, and more preferably 99% or more.

The nitrogen-rich stream removed through the carbon monoxide pressure swing adsorption process may be used as a raw material for ammonia gas synthesis. In the ammonia synthesis, the conventional Haber-Bosch process may be used, hydrogen recovered from the hydrogen recovery membrane may be used as a hydrogen raw material, and when the recovered hydrogen is insufficient for ammonia synthesis at a nitrogen:hydrogen volume ratio of 1:3, environmentally friendly green hydrogen or blue hydrogen may be additionally supplied.

In one specific example, the nitrogen stream separated through the pressure swing adsorption process may be additionally subjected to a separation step for hydrogen recovery. The separation process for hydrogen recovery includes one or more selected from an adsorption method, a low-temperature distillation method, and a membrane separation method. Preferably, a hydrogen recovery separation process using a membrane may be used, but is not limited thereto. When hydrogen is separated using a membrane, the process and operation may be simplified, modulation is easily achieved, and relatively high energy efficiency is obtained, which is effective in significantly reducing the overall process cost.

In one specific example, the hydrogen recovery membrane may separate hydrogen based on a polymer material, and the polymer material includes one or more selected from the group consisting of polysulfone, polyimide, polyphenylene oxide, polycarbonate, poly(ether imide), and cellulose acetate. The polymer material used in the membrane may be a hybrid-polymer membrane type that selectively contains inorganic materials such as zeolite and a metal-organic framework (MOF) to increase the selectivity of hydrogen.

The hydrogen membrane according to the present invention has a characteristic that it is easy to recover low-concentration hydrogen such as BFG. When hydrogen is recovered using the hydrogen membrane process, a recovery rate of hydrogen is 70% or more, preferably 80% or more, and more preferably 90% or more, and a purity of the recovered hydrogen is 80% or more, and preferably 90% or more.

According to the step (1-2) of the present invention, syngas production for supplying a hydrogen-rich (H₂-rich) gas stream may be obtained from a hydrocarbon reforming process. The hydrocarbon reforming process may be selected from steam methane reforming (SMR), partial oxidation, and autothermal reforming (ATR) reactions, and preferably, the SMR reaction capable of producing hydrogen-rich syngas as shown in the following reaction formula may be used, but is not limited thereto.

SMR: CH₄ + H₂O ↔ 3H₂ + CO, ΔH = 206 kJ/mol

According to the present invention, a mixing ratio of hydrogen/carbon monoxide in the step (1-2) has a range of 2.2 to 2.7.

The process for synthesizing an oxygenate according to the step (2) of the present invention may utilize a conventional process for synthesizing an oxygenate by mixing hydrogen/carbon monoxide/carbon dioxide through a catalytic reaction. As a catalyst for the oxygenate synthesis reaction, a Cu/ZnO/Al₂O₃-based catalyst may be used, and a catalyst having a composition made up of 52 to 58% of CuO, 22 to 27% of ZnO, and 6 to 9% of Al₂O₃ may be used, but the catalyst is not particularly limited thereto.

The oxygenate according to the present invention may be an alcohol, an ether, or a mixture thereof, and is preferably a C1 to C4 alcohol, but is not limited thereto.

In one specific example, the oxygenate synthesis reaction according to the present invention may be a methanol synthesis reaction and may proceed according to the following reaction formula.

CO + 2H₂ ↔ CH₃OH

CO₂ + 3H₂ ↔ CH₃OH + H₂O

CO₂ + CO + 5H₂ ↔ 2CH₃OH + H₂O

As a result of previous studies, it is advantageous to keep the ratio close to 1 in the H₂/(2CO+3CO₂) formula because the methanol synthesis reaction requires twice as much hydrogen as the number of carbon monoxide molecules that react and three times as much hydrogen as the number of carbon dioxide molecules. Therefore, in the present invention, the carbon monoxide gas in the step (1-1) and the syngas in the step (1-2) may be mixed to keep the stoichiometric ratio H₂/(2CO+3CO₂) at 0.85 to 1.15.

In one specific example, the oxygenate synthesis reaction according to the present invention may be ethanol, propanol, or butanol, the reaction formula for synthesizing ethanol using CO and H₂ as raw materials may proceed as follows, and propanol and butanol may also be synthesized through the same reaction pathway.

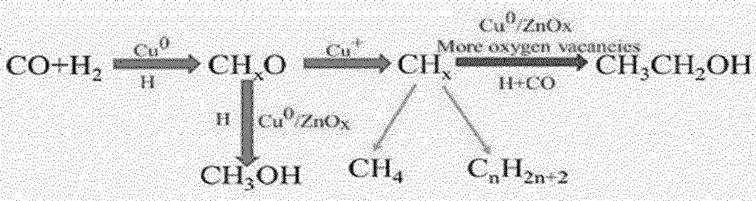

After the oxygenate synthesis reaction, the oxygenate produced by the reaction, H₂O, and unreacted gas are included. The unreacted gas may be recycled to the step (1-2) and used, and the product typically includes 10 to 30 wt% of H₂O.

Meanwhile, the olefin synthesis step according to the step (3) of the present invention may be performed by simultaneously subjecting the oxygenate produced in the step (2) and a petroleum-derived hydrocarbon to a catalytic cracking reaction. The conventional olefin production process has been performed by a steam cracking reaction of a petroleum-derived hydrocarbon or a reaction for synthesizing olefins from oxygenates such as methanol and ethanol, but there is a problem of high energy consumption. The olefin synthesis process of the present invention may utilize a catalytic process technology that combines a decomposition reaction process of petroleum-derived hydrocarbons as an endothermic reaction and an oxygenate olefin conversion process as an exothermic reaction. A circulating fluidized bed reactor is used in the olefin synthesis reaction, and for the reactor and process conditions, Korean Patent No. 10-1803406 by the same applicant may be referred to. The circulating fluidized bed reactor includes a reactor, a stripper, and a regenerator, and may use a shaped catalyst having a content of HZSM-5 of 40 wt%, but is not limited thereto.

In a specific embodiment, in the olefin synthesis process according to the present invention, olefins may be synthesized by subjecting one or more petroleum-derived hydrocarbons selected from naphtha, kerosene, and diesel and the oxygenate reactant to catalytic cracking in the presence of a catalyst.

In one specific example, the energy consumption for olefin synthesis was reduced by about 20% or more compared to the conventional technology through the simultaneous catalytic cracking reaction of the oxygenate and petroleum-derived hydrocarbon. This is because the energy generated in the exothermic reaction of the oxygenate may be converted to the decomposition reaction of hydrocarbons, which is an endothermic reaction.

Above all, according to the present invention, the oxygenate including H₂O produced in the step (2) may be used as is in the step (3) without a separate distillation purification process. In the conventional olefin synthesis reaction from the oxygenate, H₂O produced during the oxygenate synthesis is separated into a pure oxygenate through a distillation process. This is because H₂O acts as an impurity or catalyst poison in the process of producing olefins from the oxygenate. However, according to the present invention, since the decomposition reaction of the petroleum-derived hydrocarbon is an endothermic reaction that additionally supplies H₂O, the oxygenate + H₂O mixture in the step (2) may be used as is. That is, according to the present invention, since the oxygenate distillation process is omitted, the efficiency and economic feasibility of the process may be increased, and thermal neutralization of the exothermic reaction of the oxygenate and the endothermic reaction of the petroleum-derived hydrocarbon may be achieved, thereby significantly reducing energy consumption.

### [Examples]

Hereinafter, the present invention will be described in detail through Examples. However, these Examples are provided to describe the present invention in more detail, and the scope of the present invention is not limited by the following Examples.

### Examples and Comparative Examples

### (Example 1) Process of Separating BFG Gas, Synthesizing Methanol, and Producing Olefins

Blast furnace gas (BFG) includes large amounts of carbon monoxide and carbon dioxide. The BFG components of the present invention include H₂, CO₂, CO, and N₂ in amounts of 3.2%, 20.7%, 22.0%, and 54.1%, respectively. First, impurities such as dust, moisture, and sulfur present in BFG may be removed through pretreatment processes such as a filter, a condenser, and a desulfurization process.

Referring to FIG. 2, a process of preparing a raw material for plastics is performed through the steps of: separating gas from pretreated BFG, supplying hydrogen, synthesizing methanol, and producing olefins.

In the step of separating gas, carbon dioxide is first selectively separated using a membrane contactor. The membrane contactor may be miniaturized due to its simple process and has the advantages of low installation and operating costs. For the detailed structure of the membrane contactor, Korean Patent No. 10-1759101 by the same applicant may be referred to. The membrane contactor separation process is a technology in which a mixed gas and an absorbent (liquid) are brought into contact with each other using a porous hollow fiber membrane formed of a hydrophobic material, and then, carbon dioxide having high solubility is selectively absorbed into the absorbent for separation, polytetrafluoroethylene (PTFE), polypropylene (PP), polyvinylidene fluoride (PVDF), or the like may be used as the hydrophobic material, and a physical absorbent (water, methanol, dimethyl ethers of polyethylene glycol (DEPG), N-methyl-2-pyrrolidone (NMP), or the like) and a chemical absorbent (methyl diethanolamine (MDEA), diethanolamine (DEA), methyl ethanolamine (MEA), triethanolamine (TEA), or the like) may be used as the absorbent. Above all, when carbon dioxide is separated using a membrane contactor, there is no pressure drop in the main stream, and thus, repressurization and the like are not required, which is significantly advantageous in terms of energy efficiency. In addition, the separated carbon dioxide may reduce greenhouse gas emissions by carbon capture and storage (CCS) or carbon capture and utilization (CCU).

The nitrogen and carbon monoxide gases remaining after the membrane contactor process are separated using a pressure swing adsorption (PSA) device. A metal-organic framework (MOF), zeolite, activated carbon, or the like may be used as a carbon monoxide adsorbent. Hydrogen gas is included in nitrogen separated in the PSA process. The hydrogen may be selectively separated using a membrane, and a polymer membrane such as PI, PSf, PTMSP, or PTFE is used as a hydrogen membrane.

A carbon monoxide-rich gas stream is supplied to a methanol synthesis reactor together with a hydrogen-rich gas stream. At this time, the hydrogen-rich gas stream is supplied from syngas obtained from a steam reforming reaction (SMR) of methane, and a ratio of hydrogen and carbon monoxide in the SMR reaction is about 3.

The carbon monoxide-rich gas stream and the hydrogen-rich gas stream were supplied to a methanol synthesizer, and a methanol synthesis reaction was performed. A reactor filled with a CuO/ZnO/Al₂O₃ catalyst was used for methanol synthesis, and the operating conditions were maintained at a temperature of 250°C and a pressure of 50 bar.

The step of synthesizing olefins was performed by using the synthesized methanol (crude methanol, methanol:water = 80 wt%: 20 wt%) and naphtha as reactants through a simultaneous catalytic cracking reaction. A circulating fluidized bed reactor is used in the olefin synthesis reaction, and for the reactor and process conditions, Korean Patent No. 10-1803406 by the same applicant may be referred to. The circulating fluidized bed reactor includes a reactor, a stripper, and a regenerator, and a shaped catalyst having a content of HZSM-5 of 40 wt% was used as a catalyst. The naphtha and methanol as the reactants were supplied at the same weight ratio (50 wt%:50 wt%).

Table 1 shows the step-by-step gas ratio for separating gas from BFG, the raw material gas ratio supplied to the methanol synthesizer, the methanol/product ratio, and the supply ratio for olefin synthesis. The moles supplied per unit time in Table 1 were calculated based on a daily production of 100 kg of olefins.

**[Table 1]**

| Stream No. | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Pressure (bar) | 0.98 | 1 | 5 | 1 | 1 | 9 | 50 | 5 | 5 | 1.3 | 1.3 | 5 | 1 | 1 | 2.9 | 1 |
| Temperature (°C) | 24 | 100 | 38 | 38 | 38 | 38 | 208 | 25 | 152 | 331 | 25 | 120 | 35 | 25 | 110 | 0 |
| Total flow (mol/hr) | 310 | 60 | 235 | 70 | 7 | 160 | 520 | 99 | 281 | 738 | 48 | 587 | 188 | 62 | 321 | 22.89 |
| H2 | 10 | 0.1 | 7 | 0.3 | 6 | 0.7 | 340 | 0 | 0 | 543 | 0 | 0 | 0 | 0 | 34 | 0 |
| H2O | 7 | 0 | 0.1 | 0 | 0 | 0.1 | 1.8 | 0 | 0 | 101 | 0 | 146 | 36 | 0 | 36 | 0 |
| N2 | 150 | 2 | 150 | 6 | 0.6 | 140 | 7 | 0 | 0 | 543 | 0 | 0 | 0 | 0 | 0 | 0 |
| O2 | 1 | 0 | 0 | 0 | 0 | 0 | 0 | 0 | 0 | 41 | 0 | 0 | 0 | 0 | 0 | 0 |
| CO | 70 | 0.9 | 70 | 63 | 0 | 7 | 135 | 0 | 0 | 0 | 0 | 72 | 0 | 0 | 0.7 | 0 |
| CO2 | 67 | 60 | 6.7 | 0.2 | 0 | 6 | 31 | 0 | 0 | 53 | 0 | 31 | 0.7 | 0 | 0.4 | 0 |
| CH4 | 0 | 0 | 0 | 0 | 0 | 0 | 4 | 92 | 0 | 0 | 45 | 4 | 0 | 0 | 63 | 21.4 |
| CH4O | 0 | 0 | 0 | 0 | 0 | 0 | 0 | 0 | 0 | 0 | 0 | 0 | 150 | 0 | 0 | 0 |
| C2H4 | 0 | 0 | 0 | 0 | 0 | 0 | 0 | 0 | 0 | 0 | 0 | 0 | 0 | 0 | 63 | 0 |
| C2H6 | 0 | 0 | 0 | 0 | 0 | 0 | 0 | 4 | 0 | 0 | 2.1 | 0 | 0 | 0 | 23 | 1 |
| C3H6 | 0 | 0 | 0 | 0 | 0 | 0 | 0 | 0 | 0 | 0 | 0 | 0 | 0 | 0 | 45 | 0 |
| C3H8 | 0 | 0 | 0 | 0 | 0 | 0 | 0 | 1.5 | 0 | 0 | 0.7 | 0 | 0 | 0 | 8 | 0.34 |
| C4H10 | 0 | 0 | 0 | 0 | 0 | 0 | 0 | 0.6 | 0 | 0 | 0.3 | 0 | 0 | 0.4 | 1 | 0.13 |
| C5+ | 0 | 0 | 0 | 0 | 0 | 0 | 0 | 0 | 0 | 0 | 0 | 0 | 0 | 61.6 | 47 | 0 |

### (Example 2)

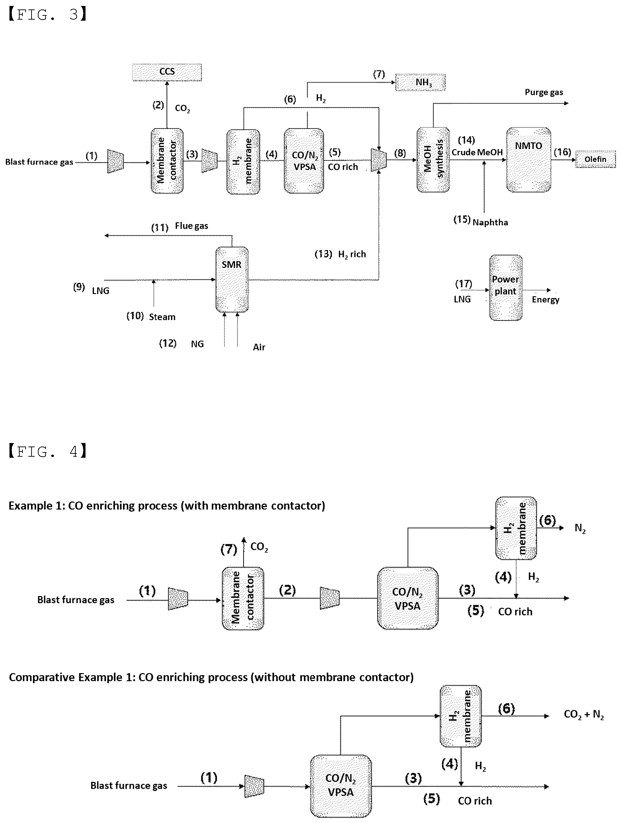

The basic process for synthesizing olefins from BFG was the same as in Example 1, except that carbon dioxide was selectively separated from blast furnace gas (BFG) using a membrane contactor, and then, hydrogen was selectively separated using a membrane before the pressure swing adsorption (PSA) device. Referring to FIG. 3, after carbon dioxide is separated from BFG, a carbon monoxide-rich stream is produced and supplied through a hydrogen membrane and a pressure circulation device.

Table 2 shows the step-by-step gas ratio for separating gas from BFG, the raw material gas ratio supplied to the methanol synthesizer, the methanol/product ratio, and the supply ratio for olefin synthesis.

**[Table 2]**

| Stream No. | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| Pressure (bar) | 0.98 | 1 | 5 | 9 | 0.5 | 1 | 9 | 50 | 5 | 5 | 1.3 | 1.3 | 5 | 1 | 1 | 2.9 | 1 |
| Temperature (°C) | 24 | 100 | 38 | 38 | 36 | 38 | 38 | 38 | 25 | 152 | 331 | 25 | 38 | 36 | 25 | 110 | 0 |
| Total flow (mol/hr) | 310 | 63 | 235 | 227 | 70 | 7 | 158 | 521 | 99 | 281 | 738 | 48 | 446 | 188 | 62 | 322 | 22.89 |
| H2 | 10 | 0.1 | 7 | 0.7 | 0 | 7 | 0.7 | 341 | 0 | 0 | 0 | 0 | 334 | 0 | 0 | 33.8 | 0 |
| H2O | 7 | 0 | 0.1 | 0.1 | 0 | 0 | 0.1 | 2 | 0 | 281 | 100 | 0 | 5 | 36 | 0 | 36 | 0 |
| N2 | 150 | 2 | 150 | 150 | 6 | 0.4 | 143 | 7 | 0 | 0 | 543 | 0 | 0 | 0 | 0 | 0 | 0 |
| O2 | 1 | 0 | 0 | 0 | 0 | 0 | 0 | 0 | 0 | 0 | 41 | 0 | 0 | 0 | 0 | 0 | 0 |
| CO | 70 | 0.9 | 70 | 70 | 62 | 0.2 | 7 | 135 | 0 | 0 | 0 | 0 | 72 | 0 | 0 | 0.7 | 0 |
| CO2 | 67 | 60 | 6 | 6 | 0.2 | 0 | 6 | 31 | 0 | 0 | 53 | 0 | 31 | 0.7 | 0 | 0.4 | 0 |
| CH4 | 0 | 0 | 0 | 0 | 0 | 0 | 0 | 4 | 92 | 0 | 0 | 45 | 4 | 0 | 0 | 63 | 21.4 |
| CH4O | 0 | 0 | 0 | 0 | 0 | 0 | 0 | 0 | 0 | 0 | 0 | 0 | 0 | 151 | 0 | 0 | 0 |
| C2H4 | 0 | 0 | 0 | 0 | 0 | 0 | 0 | 0 | 0 | 0 | | 0 | 0 | 0 | 0 | 63 | 0 |
| C2H6 | 0 | 0 | 0 | 0 | 0 | 0 | 0 | 0 | 4.3 | 0 | 0 | 2.1 | 0 | 0 | 0 | 23 | 1 |
| C3H6 | 0 | 0 | 0 | 0 | 0 | 0 | 0 | 0 | 0 | 0 | 0 | 0 | 0 | 0 | 0 | 46 | 0 |
| C3H8 | 0 | 0 | 0 | 0 | 0 | 0 | 0 | 0 | 1.5 | 0 | 0 | 0.7 | 0 | 0 | 0 | 8 | 0.34 |
| C4H10 | 0 | 0 | 0 | 0 | 0 | 0 | 0 | 0 | 0.6 | 0 | 0 | 0.3 | 0 | 0 | 0.4 | 1 | 0.13 |
| C5+ | 0 | 0 | 0 | 0 | 0 | 0 | 0 | 0 | 0 | 0 | 0 | 0 | 0 | 0 | 61.6 | 47 | 0 |

### (Comparative Example 1)

The basic process for synthesizing olefins from BFG was the same as in Example 1, except that a swing adsorption reactor (PSA) was used instead of a membrane contactor in the step of separating carbon dioxide from BFG.

The process conceptual diagrams of Example 1 and Comparative Example 1 depending on the use of the membrane contactor are illustrated in FIG. 4, and the concentrations of gas components for each process stream are summarized as shown in Table 3.

**[Table 3]**

| Gas components (Volume Percentage) | BFG (1) | | Membrane contactor (2) | | PSA (3) | | H₂ Membrane (4) | | CO Rich Gas (5) | | Off Gas (6) | | CO2 Rich Gas (7) | |
|---|---|---|---|---|---|---|---|---|---|---|---|---|---|---|
| | (a) | (b) | (a) | (b) | (a) | (b) | (a) | (b) | (a) | (b) | (a) | (b) | (a) | (b) |
| CO₂ | 18.5 | 18.5 | 2.8 | - | 0.41 | 2.89 | 0.41 | 2.9 | 0.41 | 2.9 | 4.06 | 28.9 | 95 | - |
| N₂ | 42 | 42 | 64.11 | | 9.12 | 6.5 | 9.14 | 6.5 | 9.13 | 6.5 | 91 | 65.6 | 3.2 | |
| H₂ | 2.9 | 2.9 | 3.2 | | 0.45 | 0.3 | 90 | 90 | 8.83 | 8.8 | 0.46 | 0.32 | 0.16 | |
| CO | 19.5 | 19.5 | 29.77 | | 90 | 90 | 0.44 | 0.31 | 8163 | 81.51 | 4.41 | 3.14 | 1.5 | |

Referring to Table 3, when the carbon dioxide in the blast furnace gas was separated in the membrane contactor as in Example 1 according to the present invention, the carbon monoxide pressure swing adsorption efficiency was improved, and high-purity carbon dioxide was obtained at the same time.

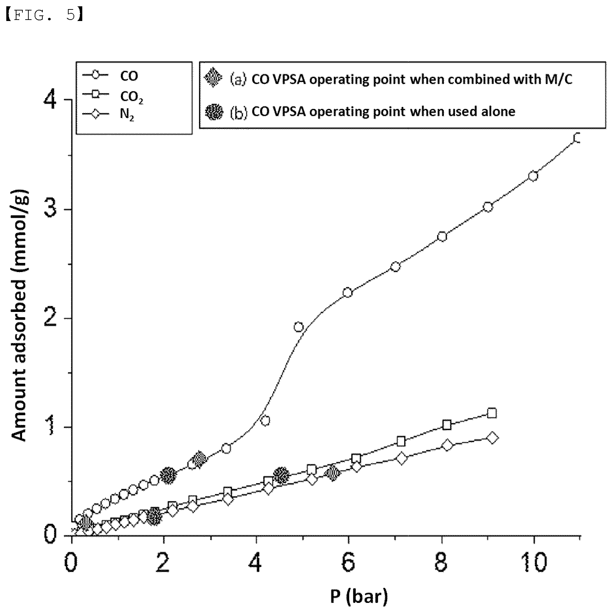

The adsorption equilibrium experimental data of Example 1 and Comparative Example 1 according to the use of the membrane contactor are illustrated in FIG. 5, and the composition by component, partial pressure, required energy, and required amount of adsorbent in the step of separating gas are summarized in Table 4.

**[Table 4]**

| | (a) Example 1 Membrane Contactor + CO VPSA | | | (b) Comparative Example 1 When using CO VPSA alone | | |
|---|---|---|---|---|---|---|
| | Composition (-) | Partial pressure (bar) | Equilibrium adsorption amount (mmol/g) | Composition (-) | Partial pressure (bar) | Equilibrium adsorption amount (mmol/g) |
| Components | | | | | | |
| -CO | 0.298 | 2.68 | 0.660 | 0.233 | 2.09 | 0.556 |
| -N₂ | 0.641 | 5.77 | 0.581 | 0.501 | 4.51 | 0.456 |
| -CO₂ | 0.029 | 0.26 | 0.031 | 0.221 | 1.99 | 0.242 |
| -H₂ | 0.032 | 0.29 | - | 0.025 | 0.22 | - |
| Purity (wf Rinse, vol%) | | 90 | | | 90 | |
| Required energy (-) | | 1.0 | | | 1.29 | |
| Amount of adsorbent required (-) | | 1.0 | | | 1.31 | |

| | | | | | | |
|---|---|---|---|---|---|---|
| *** Based on adsorption pressure of 9 bara and desorption pressure of 0.5 bara** | | | | | | |

As compared above, in the case of Example 1 in which the membrane contactor according to the present invention was applied, the carbon monoxide separation efficiency was increased compared to Comparative Example 1, and high-purity carbon dioxide was obtained at the same time. In addition, referring to Table 4, the energy required to secure the same purity of 90% increased to 129% in Comparative Example 1 compared to Example 1 in which a membrane contactor was applied, and the amount of adsorbent required to process the same raw material also increased to 131%. The present invention using a membrane contactor is significantly efficient in terms of required energy and adsorbent since offgas is used as a raw material for carbon monoxide pressure swing adsorption (PSA), and therefore, no energy is required for repressurization.

### (Comparative Example 2)

The basic process for synthesizing olefins from BFG was the same as Example 1, except that the crude methanol produced in the methanol synthesizer was not directly supplied to the olefin synthesis reaction, but was supplied to the olefin synthesis reaction through an additional distillation purification process.

### (Example 3)

The basic process for synthesizing olefins from BFG was the same as Example 1, except that water was partially purified from the crude methanol produced in the methanol synthesizer to adjust the methanol:water to 90 wt%:10 wt%.

### (Example 4)

The basic process for synthesizing olefins from BFG was the same as Example 1, except that water was partially added to the crude methanol produced in the methanol synthesizer to adjust the methanol:water to 70 wt%:30 wt%.

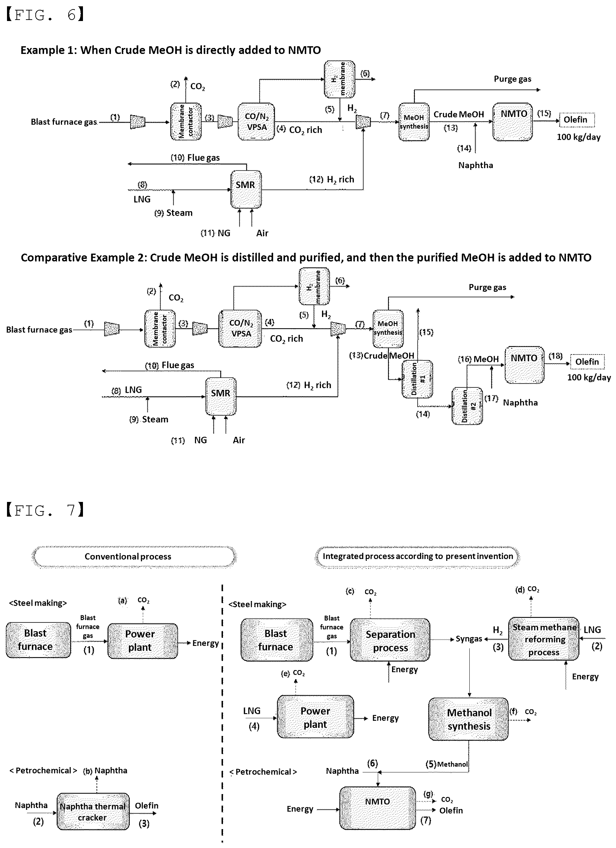

The process conceptual diagrams of Examples 1, 3, and 4, and Comparative Example 2 according to the presence or absence of the methanol additional distillation process and the water content are illustrated in FIG. 6, and the olefin yields are as shown in Table 5.

**[Table 5]**

| | Example 1 | Example 3 | Example 4 | Comparative Example 2 |
|---|---|---|---|---|
| Water content (wt%) | 20 | 10 | 30 | 0 |
| Olefin yield (%) | 42.9 | 41.3 | 44.5 | 37.7 |

According to the present invention, when crude methanol was directly supplied to a catalytic cracking reactor with naphtha at the same time to produce olefins, the olefin yield increased by 13% or more compared to the methanol additional distillation process of Comparative Example 2. This is because water in crude methanol acts as a diluent during the methanol-naphtha simultaneous catalytic cracking (naphtha-methanol to olefin: NMTO) reaction, helping to improve reactivity. As in Example 4, when the water content in crude methanol was increased to 30 wt%, the olefin yield was confirmed to increase to 44.5%.

### (Example 5)

The basic process for synthesizing olefins from BFG was the same as in Example 3, except that ethanol was synthesized instead of methanol from a carbon monoxide-rich gas stream and a hydrogen-rich gas stream.

### (Example 6)

The basic process for synthesizing olefins from BFG was the same as in Example 3, except that diesel was used instead of naphtha as the petroleum-derived hydrocarbon.

### (Example 7)

The basic process for synthesizing olefins from BFG was the same as in Example 5, except that diesel was used instead of naphtha as the petroleum-derived hydrocarbon.

The olefin yields according to the olefin synthesis raw materials of Examples 5 to 7 are summarized as shown in Table 6.

**[Table 6]**

| | Example 3 | Example 5 | Example 6 | Example 7 |
|---|---|---|---|---|
| Mixed raw material | Methanol + naphtha | Ethanol + naphtha | Methanol + diesel | Ethanol + diesel |
| Olefin yield (%) | 41.3 | 45.5 | 34 | 41.4 |

Additionally, the stream balance, utility usage, and carbon dioxide emissions of Example 1 and Comparative Example 2 depending on the direct injection of crude methanol are summarized in Table 7.

**[Table 7]**

| | | Example 1 (when adding crude MeOH) | | | Comparative Example 2 (when crude MeOH is purified by additional distillation and added to NMTO) | | | | | |
|---|---|---|---|---|---|---|---|---|---|---|
| Stream No. | | 13 | 14 | 15 | 13 | 14 | 15 | 16 | 17 | 18 |
| Pressure (bar) | | 1 | 1 | 2.9 | 1.84 | 2.05 | 1.36 | 1.16 | 1 | 2.94 |
| Tempurature (°C) | | 36 | 25 | 110 | 36 | 82 | 45 | 51 | 25 | 110 |
| Total flow (mol/hr) | | 188 | 62 | 321 | 188 | 186 | 1.12 | 150 | 62 | 284 |
| H2 | | 0 | 0 | 34 | 0 | 0 | 0 | 0 | 0 | 33 |
| H2O | | 36 | 0 | 36 | 36 | 36.37 | 0 | 0.06 | 0 | 0.06 |
| N2 | | 0 | 0 | 0 | 0 | 0 | 0 | 0 | 0 | 0 |
| O2 | | 0 | 0 | 0 | 0 | 0 | 0 | 0 | 0 | 0 |
| CO | | 0 | 0 | 0.7 | 0 | 0 | 0 | 0 | 0 | 0.72 |
| CO2 | | 0.7 | 0 | 0.4 | 0.77 | 0 | 0.77 | 0 | 0 | 0.45 |
| CH4 | | 0 | 0 | 63 | 0 | 0 | 0 | 0 | 0 | 63 |
| CH4O | | 150 | 0 | 0 | 150 | 150 | 0.35 | 150 | 0 | 0 |
| C2H4 | | 0 | 0 | 63 | 0 | 0 | 0 | 0 | 0 | 63 |
| C2H6 | | 0 | 0 | 23 | 0 | 0 | 0 | 0 | 0 | 22 |
| C3H6 | | 0 | 0 | 45 | 0 | 0 | 0 | 0 | 0 | 45 |
| C3H8 | | 0 | 0 | 8 | 0 | 0 | 0 | 0 | 0 | 7.82 |
| C4H10 | | 0 | 0.4 | 1 | 0 | 0 | 0 | 0 | 0.44 | 1.61 |
| C5+ | | 0 | 61.6 | 47 | 0 | 0 | 0 | 0 | 62 | 45 |
| Utility usage | | | | | | | | | | |
| | LP Steam(kg/hr) | - | | | 5.04 | | | | | |
| | Electricity(kW) | 2.04 | | | 2.04 | | | | | |
| | Cooling Water(kg/hr) | 591 | | | 5.19 | | | | | |
| CO₂ emissions (-) | | 1.0 | | | 1.06 | | | | | |

Referring to Table 7, when crude methanol is further purified as in Comparative Example 1 and used, as a column for purification is required, additional energy supply is required, and accordingly, carbon dioxide emissions increased by 6 to 10%.

### (Example 8) Calculation of Carbon Dioxide Reductions

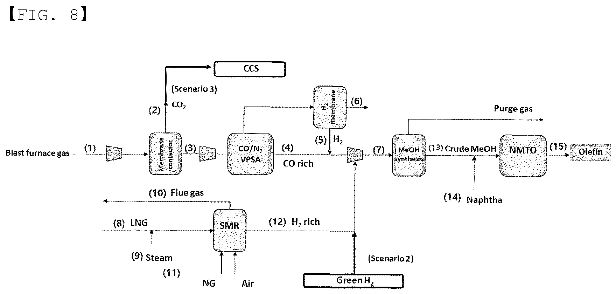

A conceptual diagram for comparing the integrated process (Example 1) for preparing a raw material for plastics from steel by-product gas according to the present invention with the conventional process in which steelmaking and petrochemical steps are separated is illustrated in FIG. 7, and the process diagrams for the process of Example 1 (Scenario 1), additional supply of renewable hydrogen (Scenario 2), and use of separated carbon dioxide for carbon capture and storage (CCS) (Scenario 3) are illustrated in FIG. 8. The carbon dioxide reduction was calculated from the carbon balance of each process and the carbon balance according to energy utility use, and the results are shown in Table 8.

**[Table 8]**

| * When utilizing 50 billion Nm³ of by-product gas per year | | | | | |
|---|---|---|---|---|---|
| **Process** | **Technology classification** | **CO₂ emissions** | **CO₂ reductions** | CO₂ reduction rate % | **Note** |
| **-** | **As-Is (based on current emissions)** | **41.2** | **-** | **-** | |
| **Scenario1 (Example 1)** | **When only BFG is utilized** | **35.4** | **5.7** | **14%** | Available to secure economic feasibility |
| **Scenario 2** | **Scenario 1 + Use of renewable H₂** | **17.6** | **23.6** | **57%** | 4.8 million tons of renewable H₂ usage |
| **Scenario 3** | **Scenario 2 + Application of CCS** | **CO₂ reduction rate of 80% or more** | | | |

Referring to Table 8, when the process of Example 1 according to the present invention was applied, carbon dioxide was reduced by 14%. In addition, when renewable hydrogen was used as a supply source in addition to Example 1, carbon dioxide was reduced by 57%, and when the separated carbon dioxide was used for CCS, the carbon dioxide reduction rate was found to be 80% or more.

As a result, when the integrated process according to the present invention was used, carbon dioxide, carbon monoxide, hydrogen, and nitrogen gases were separated from steel gas while reducing the amounts of energy and adsorbent used. In addition, according to the application of the integrated process, carbon dioxide emissions were reduced by 14%, and when the separated carbon dioxide was reused, a reduction efficiency of 80% or more was achieved. Furthermore, BFG gas was used to produce high-value-added raw materials such as olefins.

Hereinabove, although the present invention has been described by specific matters, limited embodiments, and the drawings, they have been provided only for assisting in the entire understanding of the present invention. Therefore, the present invention is not limited to the embodiments, and various modifications and changes may be made by those skilled in the art to which the present invention pertains from this description.

Therefore, the spirit of the present invention should not be limited to the described embodiments, but the claims and all modifications equal or equivalent to the claims are intended to fall within the spirit of the present invention.

## Claims

1. A method for preparing a raw material for plastics from steel by-product gas, the method comprising the steps of:
(1-1) separating carbon dioxide from a steel by-product gas stream to produce a carbon monoxide-rich (CO-rich) gas stream;
(1-2) supplying a hydrogen-rich (H₂-rich) gas stream from syngas or renewable hydrogen;
(2) receiving the carbon monoxide-rich gas stream in the step (1-1) and the hydrogen-rich gas stream in the step (1-2) and synthesizing an oxygenate; and
(3) reacting the oxygenate synthesized in the step (2) with a petroleum-derived hydrocarbon simultaneously to produce olefins,
wherein in the step (1-1), the carbon dioxide in the steel by-product gas is 1 to 50 vol% and is separated through a membrane contactor.

2. A method for preparing a raw material for plastics from steel by-product gas, the method comprising the steps of:
(1-1) separating carbon dioxide from a steel by-product gas stream to produce a carbon monoxide-rich (CO-rich) gas stream;
(1-2) supplying a hydrogen-rich (H₂-rich) gas stream from syngas or renewable hydrogen;
(2) receiving the carbon monoxide-rich gas stream in the step (1-1) and the hydrogen-rich gas stream in the step (1-2) and synthesizing an oxygenate; and
(3) reacting the oxygenate synthesized in the step (2) with a petroleum-derived hydrocarbon simultaneously to produce olefins,
wherein the oxygenate synthesized in the step (2) is introduced into the step (3) without a separate purification process to continuously produce olefins.

3. The method of claim 1 or 2, wherein the steel by-product gas is blast furnace gas (BFG), and includes 10 to 30 vol% of carbon dioxide, 10 to 30 vol% of carbon monoxide, 40 to 70 vol% of nitrogen, and 1 to 5 vol% of hydrogen.

4. The method of claim 1 or 2, wherein the oxygenate includes an alcohol, an ether, or a mixture thereof.

5. The method of claim 1 or 2, wherein the petroleum-derived hydrocarbon includes one or more selected from naphtha, gasoline, kerosene, and diesel.

6. The method of claim 1 or 2, further comprising, after the separation of the carbon dioxide in the step (1-1), a step of sequentially separating nitrogen and hydrogen or sequentially separating hydrogen and nitrogen.

7. The method of claim 6, wherein in the step of separating hydrogen, a hydrogen membrane is used.

8. The method of claim 6, wherein in the step of separating nitrogen, pressure swing adsorption (PSA) is used.

9. The method of claim 6, wherein the separated hydrogen is additionally mixed into the hydrogen-rich (H₂-rich) gas stream in the step (1-2) and reused.

10. The method of claim 1 or 2, wherein a molar ratio of H₂/(2CO+3CO₂) for methanol synthesis in the step (2) is 0.9 to 1.5.

11. The method of claim 1 or 2, wherein the carbon dioxide separated from the steel by-product gas is supplied to a carbon capture and storage (CCS) or carbon capture and utilization (CCU) process.

12. The method of claim 8, wherein the nitrogen separated by the pressure swing adsorption (PSA) is supplied to a nitrogenate production process.

13. The method of claim 1 or 2, wherein in the step (3), a composite including the oxygenate includes 10 to 30 parts by weight of moisture (H₂O).

14. The method of claim 1 or 2, wherein in the step (3), a supply ratio of the oxygenate and the petroleum-derived hydrocarbon is 0.7 to 5 parts by weight of the petroleum-derived hydrocarbon with respect to 1 part by weight of the oxygenate.

15. The method of claim 1 or 2, wherein reaction temperature and space velocity ranges in the step (3) are 500 to 700°C and 5 to 30 h⁻¹.
